# EUROPEAN PATENT SPECIFICATION

(11) **EP 1 842 531 B1**
(45) Date of publication and mention of the grant of the patent: **13.07.2011**
(21) Application number: 07006918.2
(22) Date of filing: 03.04.2007
(51) Int. Cl.: A61K 9/00, A61K 47/44, A61K 36/81

(54) **Composition containing capsicum natural extracts**
Zusammensetzung mit natürlichen Capsicumextrakten
Composition contenant des extraits naturels de capsicum

(30) Priority: 07.04.2006 IT mi20060691
(43) Date of publication of application: 10.10.2007
(73) Proprietor: PH & T S.P.A., 20145 Milano (IT)
(72) Inventor: Niccolai, Fabrizio, 20144 Milano (IT); Ciabatti, Pier Guido, 52010 Capolona (AR) (IT)
(74) Representative: Banfi, Paolo

(56) References cited:
- EP-A1- 0 646 372
- CH-A5- 690 023
- FR-A1- 2 849 992
- US-A1- 2005 019 384
- MARABINI S ET AL: "BENEFICIAL EFFECTS OF INTRANASAL APPLICATIONS OF CAPSAICIN IN PATIENTS WITH VASOMOTOR RHINITIS" EUROPEAN ARCHIVES OF OTO-RHINO-LARYNGOLOGY, SPRINGER INTERNATIONAL, BERLIN, DE, vol. 248, no. 4, 1991, pages 191-194, XP000603518 ISSN: 0937-4477
- STJAERNE P ET AL: "LOCAL CAPSAICIN TREATMENT OF THE NASAL MUCOSA REDUCES SYMPTOMS IN PATIENTS WITH NONALLERGIC NASAL HYPERREACTIVITY" AMERICAN JOURNAL OF RHINOLOGY, OCEANSIDE PUBLICATIONS, PROVIDENCE, RI, US, vol. 5, no. 4, 1 July 1991 (1991-07-01), pages 145-151, XP000603517 ISSN: 1050-6586
- WOLF G ET AL: "ÄNDERUNGEN DES NASALEN VOLUMSSTROMES NACH LOKALER APPLIKATION DES NEUROPEPTIDES SUBSTANZ-P UND VON CAPSAICIN CHANGES OF NASAL AIR FLOW AFTER TOPICAL APPLICATION OF THE NEUROPEPTIDE SUBSTANCE-P AND OF CAPSAICIN" LARYNGOLOGIE RHINOLOGIE OTOLOGIE UND IHRE GRENZGEBIETE VEREINIGT MIT MONATSSCHRIFT FÜR OHRENHEILKUNDE, THIEME, STUTTGART, DE, vol. 66, no. 8, 1987, pages 412-415, XP000603410 ISSN: 0340-1588
- DATABASE MEDLINE [Online] US NATIONAL LIBRARY OF MEDICINE (NLM), BETHESDA, MD, US April 2009 CIABATTI PIER GUIDO ET AL: 'Intranasal Capsicum spray in idiopathic rhinitis: a randomized prospective application regimen trial.' Database accession no. NLM19012048 & ACTA OTO-LARYNGOLOGICA APR 2009 LNKD- PUBMED:19012048, vol. 129, no. 4, April 2009 (2009-04), pages 367-371, ISSN: 1651-2251

## Description

The present invention relates to an oily composition containing capsicum natural extracts, suitable for nasal administration and useful as adjuvant in the treatment of hyperreactive rhinitis symptoms.

The nasal administration of substances with local beneficial effect has been known for some time and a number of products are available on the market. Researches were also carried out also with preparations based on capsaicin, the active ingredient of Capsicum (Martindale Ed. 34, page 1667; USP 27, page 317).

Capsaicin (USP 27, page 316) is a white powder with a melting point between 57° and 66°, practically insoluble in cold water and soluble in alcohol and chloroform.

Due to the poor water-solubility, capsaicin compositions for rhinological use contain relatively high amounts of alcohol, in order to keep the active ingredient in solution. This has two undesirable effects, i.e.:
- relevant amounts of alcohol are nebulized in the nasal mucosa, causing irritation;
- capsaicin is absorbed through the nasal mucosa thereby causing systemic effects.

Stjaerne P et al: American Journal of Rhinology, vol. 5, no. 4, (1991),pages 145-151, e.g. discloses that nasal mucosa hyperreactivity benefits from local application of a water based capsaicin ethanolic solution.

In the absence or with an insufficient amount of organic solvent, capsaicin precipitates, with the consequence that the administered dose is not accurate and that the dispenser orifice may become obstructed.

Object of the present invention is a liquid composition for nasal administration, containing capsicum oleoresin in an oily carrier, alcohol-free or with a low alcohol concentration - not exceeding 2% by weight - and containing an amount of total capsaicins ranging from 10 to 100 µg/ml for the treatment of hyperreactive rhinitis symptoms. The composition of the invention does not irritate the nasal mucosa and allows optimal delivery of the active ingredient to the nasal mucosa; moreover, it is stable in time and does not cause capsaicin precipitation.

In order to increase the residence-time of capsaicin within the nasal cavity, and particularly the contact time with the nasal mucosa, preparations in which capsaicin was dissolved in different oily carriers were studied. It was observed that some vegetable oils, such as sunflower, sweet almond, maize, sesam, soy, olive and peanut oil are advantageous over water-based compositions in that after nebulization they adhere to the nostrils and are not easily washed away by nasal secretions.

The capsicum oleoresin used in the composition of the invention is described in the US Pharmacopoeia (USP 27, page 318) and is standardized in the capsaicin content. It is an alcoholic extract from dried ripe fruits of *Capsicum annum* var. *minimum* and from some small fruits varieties of *C. frutescens* (Solanaceae), with dark-red colour and containing not less than 8% of total capsaicins (capsaicin, dihydrocapsaicin and nordihydrocapsaicin).

The composition can be prepared by diluting a suitable amount of capsicum oleoresin in the oily carrier, in particular sunflower, sweet almond, maize, sesam, soy, olive, peanut oil, or a combination thereof, so as to obtain a clear solution containing an amount of capsaicin ranging from 10 to 100 µg/ml. Alternatively, an amount of capsicum oleoresin that provides a final capsaicin concentration ranging from 10 to 100 µg/ml can be first diluted with 96.5% ethyl alcohol, then with a vegetable oil so as to obtain a stable micro-emulsion containing no more than 2% ethyl alcohol.

Further to the above-mentioned components, the composition of the invention may contain preservatives, anti-oxidizers, stabilizing agents, salts and buffering agents. All the excipients shall be physiologically compatible and in particular shall be suitable for nutritional or pharmaceutical use.

A further aspect of the invention relates to bottles or vials equipped with spray dispensers or nebulizers, containing the above-described composition. According to a preferred embodiment, the composition is dosed in amber glass bottles or vials equipped with nebulizers able to supply amounts ranging from 50 µl to 100 µl per spray.

### Example 1 - Preparation

Capsicum compositions containing from 10 µg/ml to 100 µg/ml capsaicin in an oily carrier (sunflower, sweet almond, maize, sesam, soy, olive and peanuts oil) were prepared and filled into bottles equipped with nebulizing pumps able to supply from 50 µl to 100 µl per spray. In detail:

1 gram of capsicum oleoresin containing 10% of total capsaicins is diluted to one litre with sweet almond oil under gentle stirring and keeping the temperature between 20°C and 30°C until homogeneity of the solution (time required about 15 minutes). The solution contains 100 µg/ml of total capsaicins and can be further diluted with sweet almond oil up to ten times using the same processing conditions to obtain a final concentration of total capsaicins as low as 10 µg/ml.

The solution at the desired concentration of total capsaicins is dosed in amber glass vials suitable to accomodate a nebulizing pump able to supply from 50 µl to 100 µl per spray.

### Example 2 - Preparation

2 grams of capsicum oleoresin containing 10% of total capsaicins is diluted to 100 ml with 96.5% ethyl alcohol under gentle stirring and keeping the temperature between 20°C and 25°C until homogeneity of the solution (time required about 5 minutes). This solution contains 2 mg/ml of total capsaicins.

This solution is diluted from 20 times to 200 times with sweet almond oil, under gentle agitation and keeping the temperature between 20°C and 30°C until homogeneity of the solution (time required about 15 minutes).

The solution at the desired concentration of total capsaicins is dosed in amber glass vials suitable to accomodate a nebulizing pump able to supply from 50 µl to 100 µl per spray.

### Example 3 - Chemical-physical characterization and stability

The compositions prepared according to examples 1 and 2 appear as a pale-yellow, acrid-taste oily liquid having approx. 50 mPascal/sec. viscosity and 0.9 g/ml density (at 25°C).

The content of capsaicin and of any degradation products was determined by HPLC, which allows to quantify nordihydrocapsaicin, capsaicin and diidrocapsaicin, as well as degradation products thereof. No degradation products were detected after storage at 25°C for a period of 20 months.

All compositions were subjected to stability studies at 4°C, 25°C and 40°C.

## Claims

1. A liquid composition containing capsicum oleoresin dissolved in an oily carrier and optionally ethyl alcohol in an amount not exceeding 2% by weight, and containing an amount of total capsaicins ranging from 10 µg/ml to 100 µg/ml, to be nasally administered for use in the treatment of hyperreactive rhinitis symptoms.

2. The composition according to claim 1, in which the oily carrier is selected from sunflower oil, sweet almond oil, maize oil, sesam oil, soy oil, olive oil and peanut oil or a mixture thereof.

3. The composition according to any one of claims 1 to 2, further containing preservatives, anti-oxidizers, stabilizing agents, salts and physiologically compatible buffering agents.

## Patentansprüche

1. Flüssige Zusammensetzung, welche in einem öligen Träger, und optional in einem 2 Gew.-% nicht übersteigenden Anteil von Ethylalkohol, gelöstes Capsicum-Oleoresin enthält, und welche einen Anteil an gesamten Capsaicinen im Bereich von 10 µg/ml bis zu 100 µg/ml enthält, zur nasalen Verabreichung zur Anwendung in der Behandlung von hyperreaktiven Rhinitis-Symptomen.

2. Zusammensetzung nach Anspruch 1, wobei der ölige Träger ausgewählt ist aus Sonnenblumenöl, Süßmandelöl, Maisöl, Sesamöl, Sojaöl, Olivenöl und Erdnussöl oder einer Mischung davon.

3. Zusammensetzung nach einem der Ansprüche 1 bis 2, ferner enthaltend Konservierungsmittel, Anti-Oxidationsmittel, Stabilisierungsmittel, Salze und physiologisch verträgliche Pufferagenzien.

## Revendications

1. Composition liquide comportant une oléorésine de capsicum dissoute dans un support huileux et optionnellement de l'alcool éthylique en une quantité n'excédant pas 2% en poids, et comportant une quantité totale de capsaïcines allant de 10 µg/ml à 100 µg/ml, pour administration nasale, pour son utilisation dans le traitement des symptômes de la rhinite hyperréactive.

2. Composition selon la revendication 1, dans laquelle le véhicule huileux est choisi parmi l'huile de tournesol, l'huile d'amande douce, l'huile de maïs, l'huile de sésame, l'huile de soja, l'huile d'olive, l'huile d'arachide ou un mélange de celles-ci.

3. Composition selon l'une quelconque des revendications 1 à 2 , comportant en outre des conservateurs, antioxydants, agents stabilisateurs, sels et tampons physiologiquement compatibles.
